# EUROPEAN PATENT APPLICATION

(11) **EP 4 512 814 A1**
(43) Date of publication of application: **26.02.2025**
(21) Application number: 23791770.3
(22) Date of filing: 12.04.2023
(51) Int. Cl.: C07F 7/12, A61L 27/16

(54) **METHOD FOR PURIFYING HYDROXYL-GROUP- AND (METH)ACRYL-GROUP-CONTAINING SILOXANE, AND HYDROXYL-GROUP- AND (METH)ACRYL-GROUP-CONTAINING SILOXANE COMPOSITION**

(30) Priority: 18.04.2022 JP 2022068236
(71) Applicant: SHIN-ETSU CHEMICAL CO., LTD., Tokyo 1000005 (JP)
(72) Inventor: OKAMURA Kaoru, Annaka-shi, Gunma 379-0224 (JP)
(74) Representative: V.O.
(86) International application number: PCT/JP2023/014903
(87) International publication number: WO 2023/204122

(57) **Abstract**

[Problem] The object of the present invention is to provide a method for producing a siloxane compound having a hydroxyl and (meth)acrylic groups with a high purity and high yield.

[Solution] A method for purifying a (meth)acrylic group-containing siloxane, comprising a step of subjecting a mixture comprising a (meth)acrylic group-containing siloxane represented by the formula (I) and impurities derived from the siloxane and/or starting compounds of the siloxane to vacuum distillation in the presence of a polymerization inhibitor having one or more nitroxyl radicals in one molecule and having a molecular weight of 160 to 2,000 to thereby remove the impurities. In the formula (I), R' is a hydrogen atom or a methyl group, L is a divalent hydrocarbon group having at least one hydroxyl group and optionally having an ether bond, and A is an organo (poly) siloxane residue.

## Description

### FIELD OF THE INVENTION

The present invention relates to a method for purifying a siloxane containing a hydroxyl and (meth)acrylic groups.

### BACKGROUND OF THE INVENTION

Various siloxane compounds are known as monomers for the production of medical materials. For example, the siloxane compound having a hydroxyl group and a (meth)acrylic group shown in the following formula, methyl bis(trimethylsiloxy) silyl propyl glycerol methacrylate (SiGMA), is particularly well known and is widely used as a monomer for ophthalmic devices.

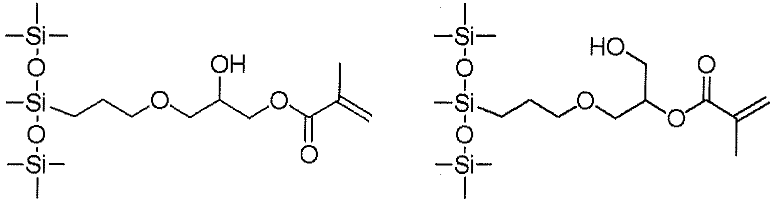

SiGMA is a monomer synthesized by the addition reaction between methacrylic acid and an epoxy-modified siloxane compound, and exhibits favorable hydrophilicity due to having hydroxyl groups in the molecule. Therefore, it has the advantage of having excellent compatibility with hydrophilic monomers such as 2-hydroxyethyl methacrylate, N,N-dimethylacrylamide, or N-vinyl-2-pyrrolidone. However, in the aforesaid production method, side reactions derived from residual epoxy-modified siloxane compounds and generated hydroxyl groups, as well as side reactions between carboxylic acid and siloxane moieties, are unavoidable. Therefore, there is a problem in which the purity of the target siloxane compound decreases.

As a means to avoid this, Patent Literature 1 describes a method for synthesizing the target product by hydrosilylation using an allyl compound having glycerol methacrylate. While this production method is an excellent technique in which residual epoxy-modified siloxane compounds are avoidable, side reactions derived from existing hydroxyl groups and (meth)acrylic groups are unavoidable. Therefore, the problem in which purity decreases remains unsolved.

Further, Patent Literature 2 describes a method for purifying SiGMA with thin-film distillation equipment (short-path distillation equipment). While this method is effective for removing polymeric materials, due to the principles of simple distillation, substances that have small boiling point differences from the target product are unable to be fractionally distilled. Further, the contamination of the product by a large amount of the polymerization inhibitor is unavoidable. That is, this method does not solve the purity problem.

### PRIOR LITERATURES

### PATENT LITERATURES

Patent Literature 1: Japanese National Phase Publication No. 2009-542674
Patent Literature 2: Japanese National Phase Publication No. 2010-510316

### BRIEF STATEMENT OF THE INVENTION

### PROBLEMS TO BE SOLVED BY THE INVENTION

As described above, siloxane compounds having hydroxyl groups and (meth)acrylic groups have excellent compatibility with hydrophilic monomers and are suitable for producing ophthalmic devices and the like.

Distillation is widely used in the chemical industry as a method for purifying compounds to high purities. However, hydroxyl group- and (meth)acrylic group-containing siloxanes have high boiling points and, therefore, polymerization of the (meth)acrylic groups in these substances is unavoidable with conventional distillation methods. Further, decreases in purity during distillation due to the transesterification reaction between hydroxyl groups and (meth)acrylic groups are unavoidable and, therefore, there is also a problem of gelation during distillation due to generated side products.

Ophthalmic devices using these siloxane compounds are known to have high oxygen permeability, but there is a demand for devices with even higher purities. Therefore, there is a demand for a method for producing a siloxane compound having a hydroxyl and (meth)acrylic groups with high purity and high yield.

The object of the present invention is to provide a method for producing a siloxane compound having a hydroxyl and (meth)acrylic groups with a high purity and high yield.

### SOLUTIONS TO THE PROBLEMS

In order to solve the aforesaid problems, the present invention has been made by finding that in a method for producing a siloxane compound having a hydroxyl group and (meth)acrylic groups, a reaction mixture comprising impurities derived from the siloxane and the starting compounds of the siloxane is vacuum distilled in the presence of a polymerization inhibitor having the specific structure shown below, thereby allowing a (meth)acrylic group-containing siloxane having the target structure to be obtained with high purity and high yield.

That is, the present invention provides a method for purifying a (meth)acrylic group-containing siloxane, comprising a step of subjecting a mixture comprising a (meth)acrylic group-containing siloxane represented by the following formula (I) and impurities derived from the siloxane and/or starting compounds of the siloxane to vacuum distillation in the presence of a polymerization inhibitor having one or more nitroxyl radicals in one molecule and having a molecular weight of 160 to 2,000 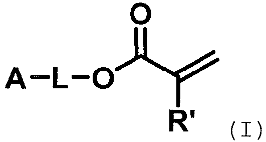 wherein, R' is a hydrogen atom or a methyl group, L is a divalent hydrocarbon group having at least one hydroxyl group and optionally having an ether bond, and A is an organo(poly)siloxane residue,
to thereby remove the impurities.

Preferably, the present invention provides a method for purifying the (meth)acrylic group-containing siloxane, wherein the polymerization inhibitor is a compound represented by the following formula (a), (a'), or (b): 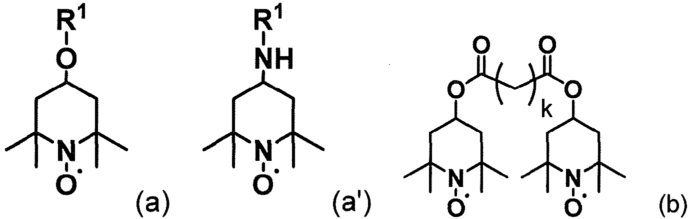 wherein R¹ is a hydrogen atom or an acyl group having 2 to 10 carbon atoms and k is an integer of 2 to 10.

The present invention further provides a composition comprising a (meth)acrylic group-containing siloxane represented by the following formula (I), 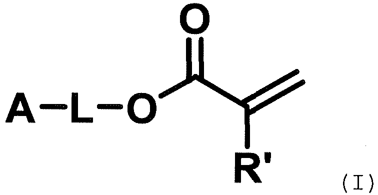 wherein R¹ is a hydrogen atom or a methyl group, L is a divalent hydrocarbon group having at least one hydroxyl group and optionally having an ether bond, and A is an organo(poly)siloxane residue,
wherein the amount of (meth)acrylic group-containing siloxane represented by formula (I) contained in the composition is 95% by mass or more, and
an amount of the following component (B), (C) or (D) in the composition is 0.5% by mass or less respectively,
   (B) a siloxane having two hydroxyl groups in one molecule
   (C) a siloxane having two (meth)acrylic groups in one molecule, and
   (D) a siloxane having one epoxy group in one molecule.

### EFFECTS OF THE INVENTION

The purification method of the present invention provides the hydroxyl group- and (meth)acrylic group-containing siloxane represented by the aforesaid formula (I) with a high purity. The hydroxyl group- and (meth)acrylic group-containing siloxane obtained by the production method of the present invention has a high purity and is suitable as a medical material such as an ophthalmic device. Further, the production method of the present invention allows the target product to be obtained with a higher yield than conventional methods. Further, the residue in the tank (stillage) after the distillation purification process is less likely to gel and, therefore, when distillation is performed in a batch operation, the transition to the next batch is made smoothly and leading to a reduction in production time.

### DETAILED DESCRIPTION OF THE INVENTION

The present invention will be described in more detail below, but not limited them.

The production method of the present invention is characterized in that the siloxane is purified by vacuum distilling a mixture comprising the (meth)acrylic group-containing siloxane represented by the aforesaid formula (I) and impurities in the presence of a polymerization inhibitor having one or more nitroxyl radicals in one molecule and having a molecular weight of 160 to 2,000. That is, the polymerization inhibitor having a specific nitroxyl radical structure prevents decreases in purity during distillation due to the transesterification reaction between hydroxyl groups and (meth)acrylic groups and prevents the polymerization of side products and (meth)acrylic groups, thereby allowing for the distillation of a hydroxyl group- and (meth)acrylic group-containing siloxane. As a result, a hydroxyl group- and (meth)acrylic group-containing siloxane is obtained with high purity and high yield. The impurities are compounds derived from the aforesaid siloxane and/or compounds derived from the starting compounds of the aforesaid siloxane, and examples thereof include a siloxane having two hydroxyl groups in the same molecule, a siloxane having two (meth)acrylic groups, and a siloxane having one epoxy group in one molecule.

In the aforesaid formula (I), R' is a hydrogen atom or methyl group. L is a divalent hydrocarbon group having at least one hydroxyl group and optionally having an ether bond, or is preferably a group selected from an alkylene group having 4 to 20 carbon atoms, or an oxyalkylene group having 4 to 20 carbon atoms that has an ether bond in the middle of the functional group chain. L has one or more hydroxyl groups in the middle of the functional group chain.

A is an organo(poly)siloxane residue, preferably a group represented by the following formula (II) or (II'). 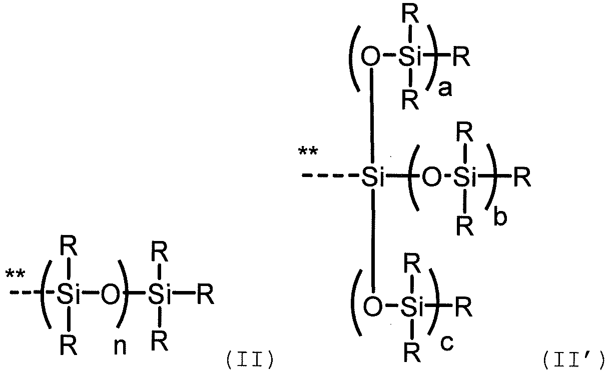 wherein R is, independently of each other, a monovalent hydrocarbon group having 1 to 6 carbons, n is an integer of 0 to 10, *a* is an integer of 0 to 10, b is an integer of 0 to 10 and c is an integer of 0 to 10, provided that a + b + c is 2 or more, the part indicated by ** is the bonding point with L in the formula (I) and the dashed line represents the bonding hand.

In the aforesaid formula (I), R' is a hydrogen atom or methyl group. Preferably, R' is a methyl group.

In the aforesaid formula (I), L is a group selected from an alkylene group having preferably 4 to 20 carbon atoms, more preferably 4 to 10 carbon atoms, or an oxyalkylene group having 4 to 20 carbon atoms, preferably 4 to 10 carbon atoms that has an ether bond in the middle of the functional group chain. L has at least one hydroxyl group

Examples thereof include any one of the following formulae (1) to (4) and (1') to (4'). 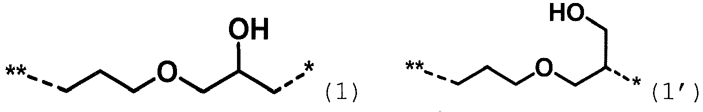 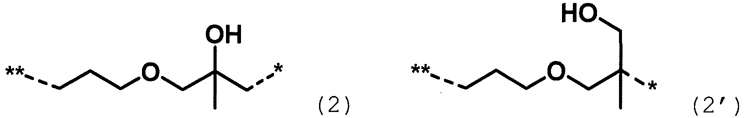 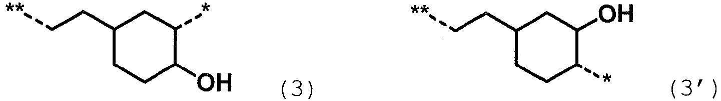 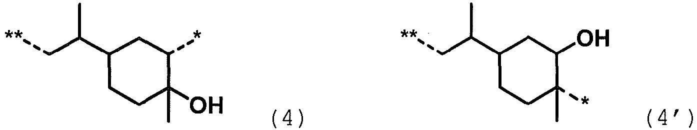 In the formulae, the part indicated by * is the bonding point with the oxygen atom in the aforesaid formula (I), the part indicated by ** is the bonding point with A in the aforesaid formula (I) and the dashed line represents the bonding hand.

In the compounds having the aforesaid formulae (1) to (4) and the compounds having the aforesaid formulae (1') to (4'), compounds having corresponding numbers indicate structural isomers. The compound obtained by the purification method of the present invention may be a mixture of these structural isomers.

In the aforesaid formulae (II) and (II'), R is, independently of each other, a monovalent hydrocarbon group having 1 to 6 carbon atoms. Examples of the monovalent hydrocarbon group include alkyl groups such as a methyl group, ethyl group, propyl group, butyl group, pentyl group, or hexyl group; cycloalkyl groups such as a cyclopentyl group or cyclohexyl group; and aryl groups such as a phenyl group. R is preferably an alkyl group having 1 to 4 carbon atoms or more preferably a methyl group or butyl group. Butyl groups include an n-butyl group, isobutyl group, sec-butyl group and t-butyl group, and an n-butyl group is particularly preferable. Preferably, in formula (II), the R bonded to the terminal silicon atom is an n-butyl group.

In the aforesaid formula (II), n is an integer of 0 to 10. n is preferably an integer of 0 to 4, or particularly preferably 4.

In the aforesaid formula (II'), a is an integer of 0 to 10, b is an integer of 0 to 10, and c is an integer of 0 to 10, with the proviso that a + b + c is 2 or more, or preferably 2 to 20. Preferably, a, b, and c are 0 to 4, or particularly preferably a is 1, b is 1, and c is 0.

The method for synthesizing the (meth)acrylic group-containing siloxane represented by the aforesaid formula (I) is not particularly limited, and can be performed according to a conventionally known method. Examples thereof include a method in which an organo (poly) siloxane having epoxy groups is reacted with acrylic acid or methacrylic acid in the presence of an alkali metal salt of acrylic acid or methacrylic acid, and a method in which a hydrogensiloxane is reacted with a (meth)acrylic group- and hydroxyl group-containing unsaturated compound.

The (meth)acrylic group- and hydroxyl group-containing unsaturated compound is preferably one that provides the structure of L described above. Examples thereof include the following compounds having at least one hydroxyl group. 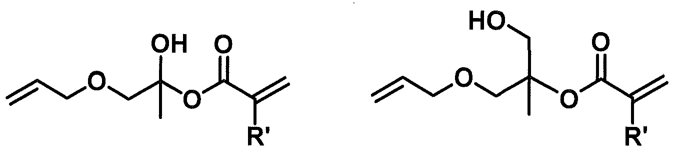 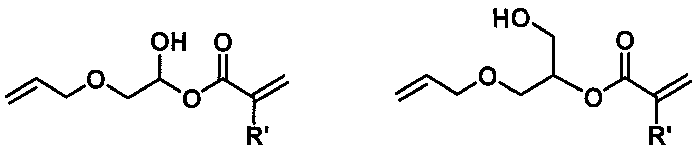 wherein R' is a hydrogen atom or a methyl group.

The hydrogen siloxane is preferably one that provides the structure of A described above. Examples thereof include a linear organohydrogen siloxane having 2 to 20 silicon atoms, preferably 1 to 12 silicon atoms, or more preferably 3 to 6 silicon atoms, and preferably having one hydrogen atom bonded to a silicon atom. Examples thereof include 1,1,1,3,5,5,5-heptamethyltrisiloxane (MHM) and 1-butyl-1,1,2,2,3,3,4,4,5,5-decamethylheptasiloxane (BD3H).

The organo(poly)siloxane containing an epoxy group may be those that give the aforesaid A-L- structure, such as the following structures.

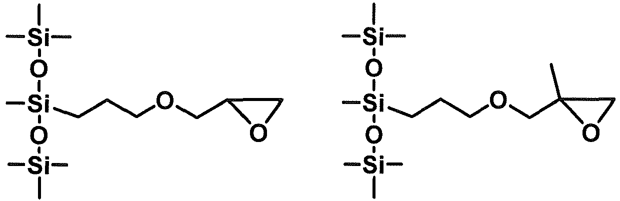

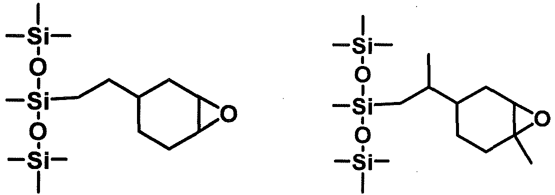

The present invention provides a method for purifying the (meth)acrylic group-containing siloxane, comprising a step of subjecting a mixture of the (meth)acrylic group-containing siloxane represented by the aforesaid formula (I) and impurities to vacuum distillation in the presence of a polymerization inhibitor having one or more nitroxyl radicals in one molecule and having a molecular weight of 160 to 2,000 to remove the impurities.

The polymerization inhibitor of the present invention is characterized by being a compound having one or more nitroxyl radicals represented by the following formula in one molecule and having a molecular weight of 160 to 2,000 or preferably 200 to 1,000. A molecular weight within this range allows the desired boiling point to be obtained. This prevents the polymerization inhibitor from being distilled off during distillation, prevents the distillate from being contaminated by the polymerization inhibitor, and effectively prevents polymerization of the stillage. If the molecular weight is too large, the solubility in the substrate may be poor, or the polymerization-inhibiting function may be poor due to a smaller equivalent of nitroxyl radicals.

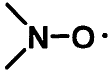

The aforesaid polymerization inhibitor is preferably a compound represented by the following formula (a), (a') or (b). 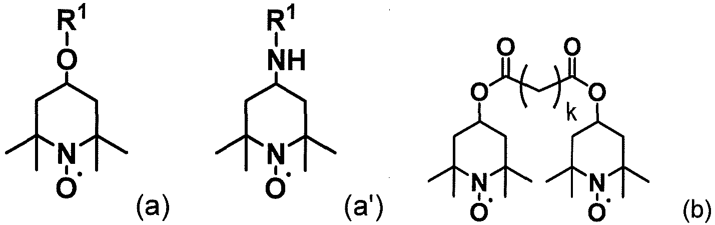 wherein R¹ is a hydrogen atom or an acyl group having 2 to10 carbon atoms and k is an integer of 2 to 10.

Examples of the acyl group for the aforesaid R¹ include saturated hydrocarbon-containing acyl groups such as an acetyl group, propionyl group, butyryl group, or isobutyryl group, and aromatic acyl groups such as a benzoyl group. The number of carbon atoms in the acyl group includes the number of carbonyl carbon atoms. R¹ is preferably a hydrogen atom, acetyl group, or benzoyl group.

k in the aforesaid formula is preferably 4 to 8, or more preferably 6 to 8.

The polymerization inhibitor of the present invention is, more specifically, a compound including the 2,2,6,6-tetramethylpiperidine-1-oxyl residue represented by the aforesaid formula (a). More specific examples thereof include 4-acetamido-2,2,6,6-tetramethylpiperidine-1-oxyl (molecular weight: 213.30), 4-benzoyloxy-2,2,6,6-tetramethylpiperidine-1-oxyl (molecular weight: 276.36), 4-hydroxy-2,2,6,6-tetramethylpiperidine-1-oxyl (molecular weight: 172.25), and a compound represented by the following formula (5) (molecular weight: 510.72), which is a product of a condensation reaction (esterification) between 4-hydroxy-2,2,6,6-tetramethylpiperidine-1-oxyl and 1,8-octanedicarboxylic acid.

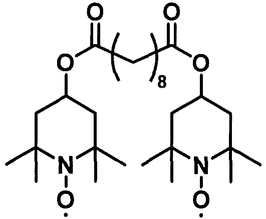

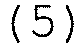

In particular, 4-hydroxy-2,2,6,6-tetramethylpiperidine-1-oxyl (molecular weight: 172.25), 4-benzoyloxy-2,2,6,6-tetramethylpiperidine-1-oxyl (molecular weight: 276.36), and the compound represented by the aforesaid formula (5) are preferable. The compound represented by the formula (5) is more preferable.

The aforesaid compound may be added singly or in combination of two or more types. The amount of polymerization inhibitor may be any concentration at which the polymerization inhibitor having nitroxyl radical dissolves in the composition solution comprising the hydroxyl group- and (meth)acrylic group-containing siloxane. From the viewpoint of cost, the amount of polymerization inhibitor is, in the solution comprising the hydroxyl group- and (meth)acrylic group-containing siloxane, 10 to 10,000 ppm, preferably 50 to 5,000 ppm, or more preferably 100 to 2,000 ppm, based on the total mass of the hydroxyl group- and (meth)acrylic group-containing siloxane. If the amount is lower than the aforesaid concentration, the polymerization-inhibiting effect may be weak, resulting in gelation or the like may occur during the distillation process. If the amount is higher than the aforesaid concentration, precipitation may occur within the distillation tower, cleaning the inside of the tower may be required, which is industrially disadvantageous and thus not preferable.

The aforesaid polymerization inhibitor may be used in combination with other conventionally known polymerization inhibitors. Examples thereof include: hindered phenol-based polymerization inhibitors such as 4,4'-butylidenebis(6-t-butyl-m-cresol), 2,2'-methylenebis(4-methyl-6-t-butylphenol), 2,2'-methylenebis(4-ethyl-6-t-butylphenol), 2,6-di-t-butyl-p-cresol, or 2,t-butyl-hydroxyanisole; and hydroquinone-based polymerization inhibitors such as 2,5-dit-amyl-hydroquinone, 2,5-di-t-butyl-hydroquinone, 2-t-butyl-hydroquinone, or hydroquinone monomethyl ether.

Amine-based polymerization inhibitors such as diphenylamine, N-nitroso-N-phenylhydroxylamine, N-nitroso-N-phenylhydroxylamine ammonium salt, or N-nitroso-N-phenylhydroxylamine aluminum salt; phenothiazine-based polymerization inhibitors such as phenothiazine; and metal-based polymerization inhibitors such as copper dimethyldithiocarbamate or N-nitroso-N-phenylhydroxylamine aluminum salt may promote transesterification during the distillation process of a hydroxyl group- and (meth)acrylic group-containing siloxane and, therefore, these are not preferable.

The vacuum distillation of the present invention can be performed according to a conventionally known method. For example, the conventionally known method is only required to be a rectification using a distillation tower or a short-path distillation such as a thin film distillation. The thin-film distillation may be performed just once, or may be performed multiple times under the same or different conditions. For the vacuum distillation, rectification is preferable from the viewpoint of the number of distillation stages. With a simple distillation such as a short-path distillation, the separation of the target product may be insufficient and a preferred purity may not be obtained. Two or more types of these distillation methods may be used in combination.

The temperature for the vacuum distillation is preferably 50°C to 250°C, or more preferably 100°C to 220°C. If the distillation is conducted at a temperature lower than the lower limit above, the pressure needs to be lowered, which may cause the vacuum equipment to be contaminated by impurities, reducing the vacuum level and, therefore, it is not preferable. It is preferable to set the temperature during the fractional distillation of the impurities and the temperature during the fractional distillation of the target product stepwise within the aforesaid range. In particular, it is preferable to conduct the fractional distillation of the compound represented by the aforesaid formula (1) at a temperature of 160°C or more, or preferably 160°C or more and 250°C or less. If the distillation is conducted at a temperature higher than the aforesaid upper limit, the purity may decrease due to the transesterification of the hydroxyl group- and (meth)acrylic group-containing siloxane or gelation may occur due to thermal polymerization, and is thus not preferable.

The pressure for the vacuum distillation may be changed as desired, but is preferably 666.6 Pa (5 Torr) or less, or more preferably 266.6 Pa (2 Torr) or less. If the distillation is performed at a pressure higher than the aforesaid pressure, heating at a high temperature is required to vaporize the target product, which may cause the purity to decrease due to the transesterification above or may cause gelation due to thermal polymerization, and is thus not preferable.

During vacuum distillation, bubbling using a gas may be performed if necessary. Bubbling prevents vaporized materials from remaining. Gases such as nitrogen, oxygen, argon, or air may be used. A gas including oxygen is preferable, and a polymerization-inhibiting effect due to the oxygen is expected.

The purification method of the present invention allows the target hydroxyl group- and (meth)acrylic group-containing siloxane to be obtained with high purity. The hydroxyl group- and (meth)acrylic group-containing siloxane having a high purity stabilizes the quality of the polymer when polymerized with other monomers, and also prevents unreacted materials from remaining after polymerization. This can streamline the synthesis and purification processes, which were difficult with conventional siloxanes. The obtained purity of the (meth)acrylic group-containing siloxane is preferably 95% by mass or more, or more preferably 96% by mass or more. The obtained purity of the (meth)acrylic group-containing siloxane of the present invention may include structural isomers represented by the aforesaid formula (I) in which the structure of L is different. For example, the compound having the structure of L represented by the afore-described formula (1) and the compound having the structure of L represented by the afore-described formula (1') are, in total, preferably 95% by mass or more, or more preferably 96% by mass or more.

The hydroxyl group- and (meth)acrylic group-containing siloxane obtained by the purification method of the present invention is characterized by the presence of few siloxanes having two hydroxyl groups or two (meth)acrylic groups in the same molecule. The siloxanes having two (meth)acrylic groups in the same molecule become crosslinking components during polymerization, which may cause an unexpected increase in molecular weight. The siloxanes having two hydroxyl groups in the same molecule become unreacted materials during polymerization. Preferably, an amount of each of these siloxanes is, based on the total mass of the (meth)acrylic group-containing siloxane and these siloxanes, 0.5% or less, or more preferably 0.3% or less.

The hydroxyl group- and (meth)acrylic group-containing siloxane obtained by the purification method of the present invention is characterized by the presence of few epoxy group-containing siloxanes. The epoxy group-containing siloxane is derived from unreacted materials during the condensation reaction with (meth)acrylic acid. Preferably, an amount of the epoxy group-containing siloxane is, based on the total mass of the (meth)acrylic group-containing siloxane and epoxy group-containing siloxane, 0.5% or less, or more preferably 0.3% or less.

That is, the present invention provides a composition comprising a (meth)acrylic group-containing siloxane represented by the following formula (I), 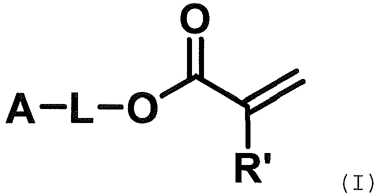 wherein R' is a hydrogen atom or a methyl group, L is a divalent hydrocarbon group which has at least one hydroxyl group and may have an ether bond, and A is an organo(poly)siloxane residue,
wherein the amount of (meth)acrylic group-containing siloxane represented by formula (I) contained in the composition is 95% by mass or more, and
an amount of the following component (B), (C) or (D) in the composition is 0.5% by mass or less respectively,
   (B) a siloxane having two hydroxyl groups in one molecule
   (C) a siloxane having two (meth)acrylic groups in one molecule, and
   (D) a siloxane having one epoxy group in one molecule.

In particular, the amount of component (D) in the composition is preferably 0% by mass.

The compound obtained by the method of the present invention contains few of the impurities described above and, thereby, the target hydroxyl group- and (meth)acrylic group-containing siloxane product is suitably used as a medical material such as an ophthalmic device. The amount (%) of these impurities refers to the area percentage of the chromatogram determined by gas chromatography analysis with conditions described after in below.

The (meth)acrylic group-containing siloxane represented by the aforesaid formula (I) obtained by the method of the present invention may be copolymerized with other monomers to produce a copolymer. Examples of other monomers include: acrylic-based monomers such as (meth)acrylic acid, methyl (meth)acrylate, ethyl (meth)acrylate, polyalkylene glycol mono(meth)acrylate, polyalkylene glycol monoalkyl ether (meth)acrylate, trifluoroethyl (meth)acrylate, 2-hydroxyethyl (meth)acrylate, or 2,3-dihydroxypropyl (meth)acrylate; acrylic acid derivatives such as N,N-dimethylacrylamide, N,N-diethylacrylamide, N-acryloylmorpholine, or N-methyl (meth)acrylamide; N,N-dimethylformamide; other unsaturated aliphatic or aromatic compounds such as crotonic acid, cinnamic acid, or vinylbenzoic acid; and polymerizable group-containing silicone compounds. Acrylic-based monomers and acrylic acid derivatives are preferable. The silicone of the present invention is polymerized with one or more compounds selected from the aforesaid other monomers to provide a polymer.

In the production of a copolymer comprising repeating units derived from the silicone of the present invention and other polymerizable monomers, the blending amount of the silicone of the present invention is, based on a total 100 parts by mass of the silicone of the present invention and other polymerizable monomers, preferably 1 to 70 parts by mass, or more preferably 10 to 60 parts by mass. The silicone of the present invention may also be homopolymerized to form a polymer.

The copolymerization of the compound of the present invention with the other polymerizable monomers may be conducted according to a conventionally known method. For example, the copolymerization may be conducted with a known polymerization initiator such as a thermal polymerization initiator or photopolymerization initiator. Examples of the polymerization initiator include 2-hydroxy-2-methyl-1-phenyl-propan-1-one, azobisisobutyronitrile, azobisdimethylvaleronitrile, benzoyl peroxide, tert-butyl hydroperoxide, and cumene hydroperoxide. These polymerization initiators may be used singly or in combination of two or more types. The blending amount of the polymerization initiator is, relative to a total 100 parts by mass of the polymerization components, 0.001 to 2 parts by mass, or preferably 0.01 to 1 part by mass.

The copolymer comprising repeating units derived from the compound of the present invention is suitable for producing ophthalmic devices such as contact lenses (e.g., hydrophilic contact lenses and silicone hydrogels), intraocular lenses, and artificial corneas. The method for producing an ophthalmic device using the polymer is not particularly limited, and may be in accordance with a conventionally known method for producing an ophthalmic device. For example, when forming the polymer into a lens shape such as a contact lens or intraocular lens, a cutting method or molding method may be used.

### EXAMPLES

The present invention will be explained below in further detail with reference to a series of the Examples and the Comparative Examples, though the present invention is in no way limited by these Examples.

The analytical methods used in the following examples and comparative examples are as follows:
a. Gas chromatography (GC) analysis
   i. Equipment and parameters
      Equipment: Agilent 7890A GC System
      Detector: Flame ionization detector (FID)
      Column: J&W HP-5MS (0.25 mm x 30 m x 0.25 µm)
      Carrier gas: Helium
      Constant flow rate: 1.0 mL/min
      Sample injection volume: 1.0 µL
      Split ratio: 50:1
      Injection port temperature: 250 °C
      Detector temperature: 300 °C
   ii. Temperature program
      Initial temperature: 50 °C
      Start time: 2 minutes
      Gradient: 10 °C/min
      Final temperature: 300 °C (holding time: 15 minutes)
   iii. Sample preparation
      The sample was diluted to 5% by mass with acetone and placed in a GC vial.
b. ¹H-NMR analysis
   i. Equipment and parameters
      Equipment: JEOL JNM-ECS400
      Pulse acquisition time: 2.1 seconds
      Pulse relaxation time: 5 seconds
      Number of accumulations: 256
   ii. Sample preparation

The sample was diluted to 10% by mass with deuterated chloroform and placed in an NMR tube with an inner diameter of 5 mm.

The compounds used in the following Examples and Comparative Examples are as follows.
AHPM: A mixture of structural isomers represented by the following formula (a) or (b), wherein a ratio of compound (a) : compound (b) is 80 : 20. 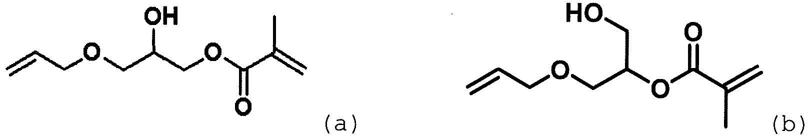
MHM: 1,1,1,3,5,5,5-Heptamethyltrisiloxane
BD3H: 1-Butyl-1,1,2,2,3,3,4,4,5,5-decamethylheptasiloxane

The siloxanes containing epoxy groups are the siloxanes represented by the following formulae (6) to (9).

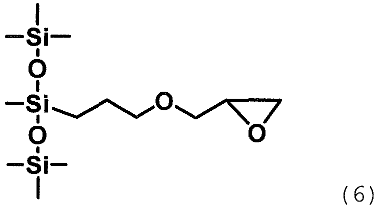

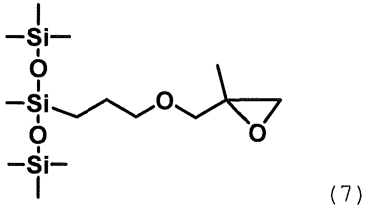

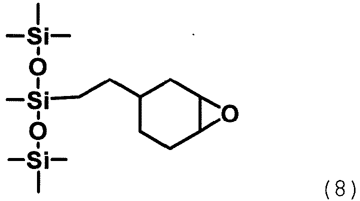

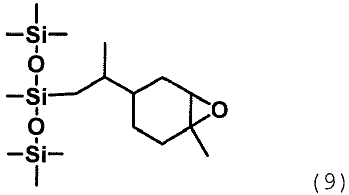

The polymerization inhibitors used in the following Examples and Comparative Examples are as follows.

Polymerization inhibitor 1: Polymerization inhibitor represented by the following formula (5), wherein Mw is 510.72 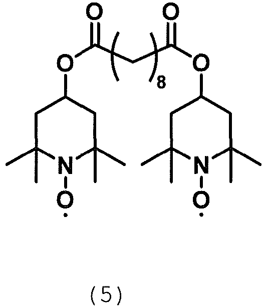
Polymerization inhibitor 2: 4-Acetamido-2,2,6,6-tetramethylpiperidine-1-oxyl, wherein Mw is 213.30, Am-TEMPO
Polymerization inhibitor 3: 4-Benzoyloxy-2,2,6,6-tetramethylpiperidine-1-oxyl, Mw is 276.36, Bz-TEMPO
Comparative Polymerization inhibitor 4: 2,6-Di-t-butyl-p-cresol, BHT
Comparative Polymerization inhibitor 5: Low-volatile phenolic Polymerization inhibitor, Sumilizer GA-80, GA-80, ex Sumitomo Chemical,
Comparative Polymerization inhibitor 6: 4-Methoxy-1-naphthol, 4-MN
Comparative Polymerization inhibitor 7: 2,2,6,6-tetramethylpiperidine-1-oxyl, Mw=156.25, TEMPO
Comparative Polymerization inhibitor 8: N-nitroso-N-phenylhydroxylamine aluminum salt, Q1301
Comparative Polymerization inhibitor 9: Copper(II) Dimethyldithiocarbamate, TTCU
Comparative Polymerization inhibitor 10: Phenothiazine

### [Synthesis Example 1]

In a 5,000-mL three-neck round-bottom flask equipped with a Dimroth condenser and thermometer, were added 1,000 g of MHM, 1,350 g of AHPM, and 0.6 g of a toluene solution of a complex of vinylsiloxane and chloroplatinic acid neutralized with sodium bicarbonate (containing 0.5 wt% of platinum), and heated to 100°C, and aged for 6 hours. After reacting, 1,200 g of n-hexane were added, and washing was performed eight times with an aqueous methanol solution (methanol : water = 4:1). The solvent was then distilled off in a vacuum at an internal temperature of 80°C, and a colorless, transparent liquid was obtained. The obtained product is a reaction mixture comprising the two types of structural isomers represented by the following formula (10). The yield was 1,230 g.

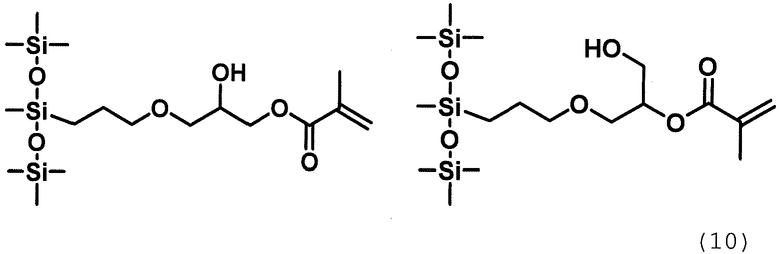

### [Synthesis Example 2]

The processes for Synthesis Example 1 were repeated except that the MHM in Synthesis Example 1 was replaced with BD3H, and a reaction mixture comprising two types of structural isomers represented by the following formula (11) was obtained.

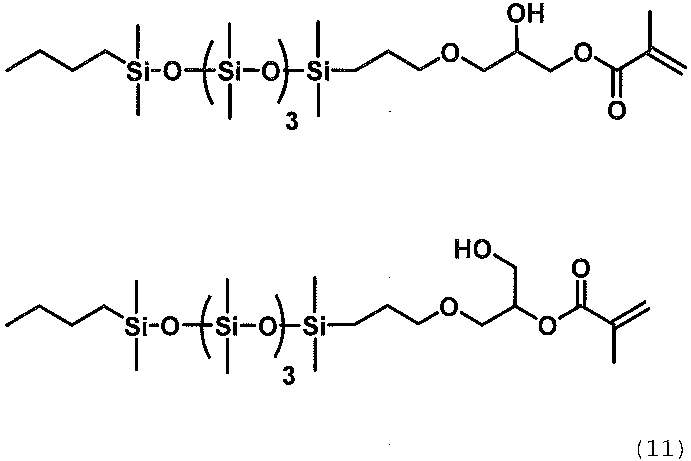

### [Synthesis Example 3]

In a 5,000-mL three-neck round-bottom flask equipped with a Dimroth condenser and thermometer, were added 1,000 g of the epoxy group-containing siloxane represented by the aforesaid formula (6), 757 g of methacrylic acid, and 96 g of sodium methacrylate, and heated to 80°C. The reaction was monitored using GC, aging was stopped when the amount of the compound represented by the aforesaid formula (6) was 2% or less based on the amount of the reaction mixture comprising the two types of structural isomers represented by the aforesaid formula (10) and, then, the reaction mixture was cooled to room temperature. After reacting, 1,000 g of n-hexane were added, then washing was performed six times with a 4% aqueous sodium hydroxide solution and three times with pure water. The solvent was then distilled off in a vacuum at an internal temperature of 80°C, and a colorless, transparent liquid was obtained. The obtained product is a reaction mixture comprising the two types of structural isomers represented by the aforesaid formula (10). The yield was 1,310 g.

### [Synthesis Example 4]

The processes for Synthesis Example 3 were repeated except that, on monitoring the reaction of Synthesis Example 3 using GC, aging was stopped when the amount of the epoxy group-containing siloxane represented by the aforesaid formula (6) was 0.1% or less based on the reaction mixture comprising the two types of structural isomers represented by the formula (10). As a result, a colorless, transparent liquid was obtained. The obtained product is a reaction mixture comprising the two types of structural isomers represented by the aforesaid formula (10).

### [Synthesis Example 5]

The processes for Synthesis Example 3 were repeated except that the epoxy group-containing siloxane in Synthesis Example 3 was replaced with the epoxy group-containing siloxane represented by the aforesaid compound (7), and a reaction mixture (colorless, transparent liquid) comprising two types of structural isomers represented by the following formulae was obtained.

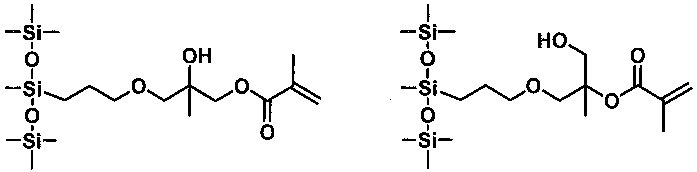

### [Synthesis Example 6]

The processes for Synthesis Example 3 were repeated except that the epoxy group-containing siloxane in Synthesis Example 3 was replaced with the epoxy group-containing siloxane represented by the aforesaid compound (8), and a reaction mixture (colorless, transparent liquid) comprising two types of structural isomers represented by the formulae below was obtained.

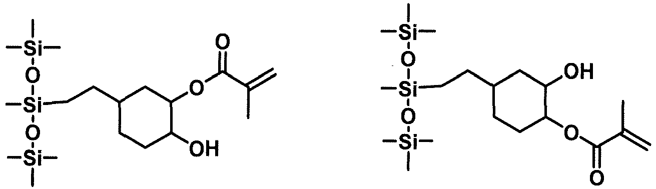

### [Synthesis Example 7]

The processes for Synthesis Example 3 were repeated except that the epoxy group-containing siloxane in Synthesis Example 3 was replaced with the epoxy group-containing siloxane represented by the aforesaid compound (9), and a reaction mixture (colorless, transparent liquid) comprising two types of structural isomers represented by the following formulae was obtained.

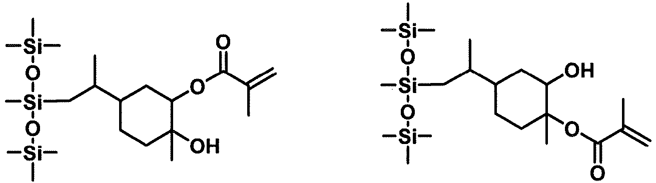

The reaction mixtures obtained in Synthesis Examples 1 to 7 were mixtures of the target compound above, the epoxy group-containing siloxane starting material, and/or the compounds represented by the following formulae (12) to (20) (hereinafter referred to as impurities). The results of GC analyses of the reaction mixtures synthesized in the Synthesis Examples are shown in Table 1 below. The ratios of contained impurities in each reaction mixture are as shown in Table 1 below. In Table 1, the purity of the target compound is the total percentage by mass of structural isomers with different structures of L in the aforesaid formula (I), based on the total mass of the reaction mixture.

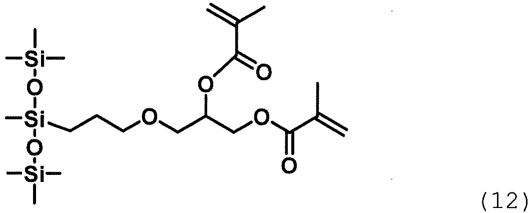

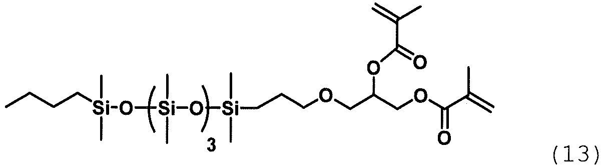

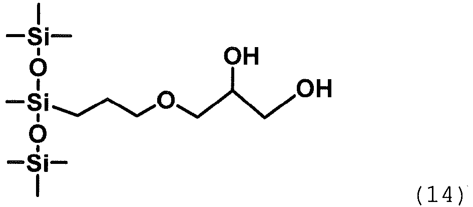

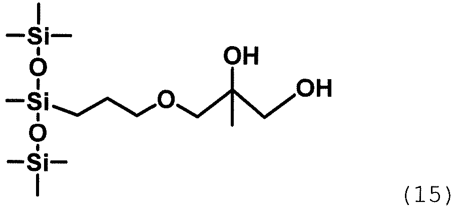

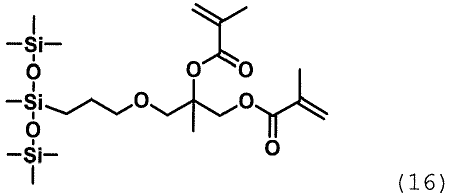

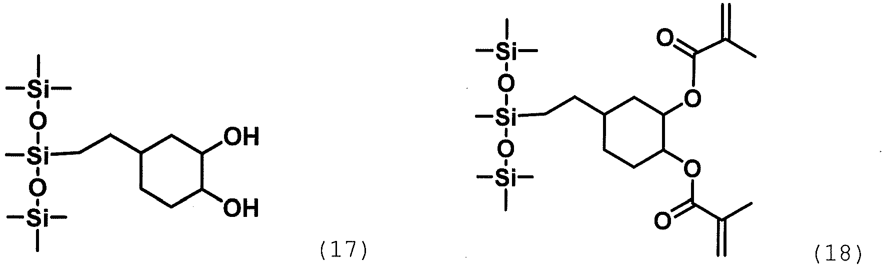

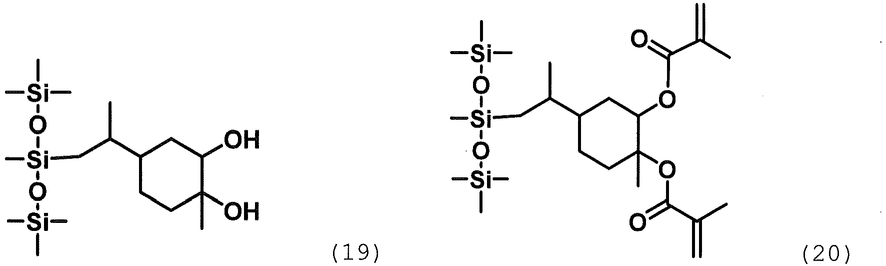

**[Table 1]**

| | Purity of the target compou nd, % | Siloxane with an epoxy group, % | | Siloxane with two hydroxyl groups, % | | Siloxane with two (meth)acrylic groups, % | |
|---|---|---|---|---|---|---|---|
| | | Struct ure | Contai ned amount , % | Struct ure | Contai ned amount , % | Struct ure | Contai ned amount ,% |
| Synthe sis Exampl e 1 | 87.7 | - | 0 | - | 0 | Formul a (12) | 0.6 |
| Synthe sis Exampl e 2 | 90.1 | - | 0 | - | 0 | Formul a (13) | 0.6 |
| Synthe sis Exampl e 3 | 89.2 | Compou nd (6) | 1.9 | Formula (14) | 0.2 | Formula (12) | 0.5 |
| Synthe sis Exampl e 4 | 87.7 | Compou nd (6) | 0.1 | Formul a (14) | 0.2 | Formul a (12) | 1.6 |
| Synthe sis Exampl e 5 | 92.3 | Compou nd (7) | 2.0 | Formul a (15) | 0.2 | Formul a (16) | 0.3 |
| Synthe sis Exampl e 6 | 90.0 | Compou nd (8) | 1.8 | Formul a (17) | 0.2 | Formul a (18) | 0.5 |
| Synthe sis Exampl e 7 | 91.1 | Compou nd (9) | 2.0 | Formul a (19) | 0.2 | Formul a (20) | 0.4 |

### [Example 1]

The polymerization inhibitor represented by the aforesaid formula (5) was dissolved in 1,000 g of the reaction mixture obtained in Synthesis Example 1 at a concentration of 1,000 ppm. Vacuum distillation was performed under the following conditions using distillation equipment with the number of theoretical plates of 5, in which the distillation tower was packed with Dixon packing material (SUS304). After the vacuum distillation, the state of the tank residue was observed after the reaction mixture was cooled to room temperature, and evaluated using the following indices. The results are shown in Table 2.
A: Fluid
B: Partially fluid (semi-gelled)
C: No fluidity (completely gelled)

### Condition 1 (pre-distillation)

Compounds with a lower boiling point than the target product were fractionally distilled.

The conditions were as follows:
Vacuum level: 66 Pa to 400 Pa;
Temperature at the top of the tower: 20°C to 180°C;
Solution temperature: 200°C or less;
Reflux ratio: 1;
Distillation time: 2 to 4 hours

### Condition 2 (main distillation)

The target product was obtained with the main distillation.

The conditions were as follows:
Vacuum level: 26 Pa to 266 Pa;
Temperature at the top of the tower: 160°C to 200°C;
Solution temperature: 250°C or less;
Completely distilled off;
Distillation time: 6 to 10 hours

For Examples 2 to 10 and Comparative Examples 1 to 8, Example 1 was repeated except that the compound and polymerization inhibitor shown in Table 2 below were used, and the siloxane compounds having (meth)acrylic groups were purified.

In Tables 2 and 3, purity is the total percentage by mass of structural isomers with different structures of L in the aforesaid formula (I) based on the total mass of the target compound obtained in the main distillation above.

**[Table 2]**

| Ex. | Reaction mixture | Polymerization inhibitor | | Purity, | Impurity, % | | | Tank residue | Distillation yields, % |
|---|---|---|---|---|---|---|---|---|---|
| | | Type | Concentration, ppm | | Siloxane with an epoxy group | Siloxane with two hydroxyl groups | Siloxane with two (meth)acrylic groups | | |
| 1 | Synthesis Example 1 | Formula (5) | 1000 | 95.2 | 0 | 0.1 | 0.2 | A | 72 |
| 2 | Synthesis Example 2 | Formula (5) | 1000 | 95.5 | 0 | 0.1 | 0.2 | A | 73 |
| 3 | Synthesis Example 3 | Formula (5) | 500 | 96.3 | 0 | 0 | 0.1 | A | 76 |
| 4 | Synthesis Example 3 | Formula (5) | 2000 | 96.5 | 0 | 0 | 0 | A | 70 |
| 5 | Synthesis Example 3 | Am-TEMPO | 2000 | 96.1 | 0.1 | 0.1 | 0.1 | B | 67 |
| 6 | Synthesis Example 3 | Bz-TEMPO | 1000 | 96.3 | 0 | 0 | 0.1 | A | 67 |
| 7 | Synthesis Example 4 | Formula (5) | 1000 | 95.8 | 0 | 0 | 0.3 | A | 70 |
| 8 | Synthesis Example 5 | Formula (5) | 1000 | 96.8 | 0.1 | 0 | 0 | A | 71 |
| 9 | Synthesis Example 6 | Formula (5) | 1000 | 95.6 | 0 | 0.2 | 0.1 | A | 71 |
| 10 | Synthesis Example 7 | Formula (5) | 1000 | 95.2 | 0.1 | 0 | 0 | A | 70 |

**[Table 3]**

| Com. Ex. | Reaction mixture | Polymerization inhibitor | | Purity, % | Impurity, % | | | Tank residue | Distillation yields, % |
|---|---|---|---|---|---|---|---|---|---|
| | | Type | Concentration, ppm | | Siloxane with an epoxy group | Siloxane with two hydroxyl groups | Siloxane with two (meth) acrylic groups | | |
| 1 | Synthesis Example 3 | BHT | 2000 | 88.2 | 0.9 | 0.2 | 0.2 | C | 17 |
| 2 | Synthesis Example 3 | BHT | 50000 | 86.6 | 0.1 | 0.6 | 0.6 | C | 46 |
| 3 | Synthesis Example 3 | GA-80 | 1000 | 90.0 | 0.8 | 0.6 | 0.6 | C | 14 |
| 4 | Synthesis Example 3 | 4-MN | 1000 | 89.5 | 0.7 | 0.2 | 0.5 | C | 20 |
| 5 | Synthesis Example 3 | TEMPO | 2000 | 94.1 | 0.4 | 0 | 0.3 | C | 34 |
| 6 | Synthesis Example 3 | Q1301 | 1000 | 90.3 | 0 | 2.8 | 3.2 | C | 58 |
| 7 | Synthesis Example 3 | TTCU | 1000 | 90.5 | 0 | 2.8 | 2.8 | C | 30 |
| 8 | Synthesis Example 3 | Phenothiazine | 1000 | 92.2 | 0.1 | 2.1 | 2.4 | B | 56 |

As shown in Table 3, in the purifications of Comparative Examples 1 to 4 using a phenol-based polymerization inhibitor, the polymerization of siloxane during vacuum distillation was unavoidable, the purity of the obtained product was low, the distillation yield was also low, and the tank residue gelled. In the purification of Comparative Example 5 using TEMPO, since TEMPO was distilled off during the vacuum distillation, the polymerization-inhibiting function was insufficient, the purity of the obtained product was low, the distillation yield was also low, and the tank residue gelled .

In the purifications of Comparative Examples 6 to 8 using a metal-based or amine-based polymerization inhibitor, since the polymerization inhibitor promoted the transesterification reaction, not only did the purity of the mixture decrease, but the amount of siloxanes having two hydroxyl groups or (meth)acrylic groups in the same molecule increased and the purity of the obtained product became low.

In contrast, as shown in Examples 1 to 10 in Table 2, purification using the polymerization inhibitor of the present invention effectively prevents the polymerization of siloxane during vacuum distillation, and vacuum distillation can be performed without gelling the tank residue. Further, since the polymerization inhibitor does not promote side reactions such as transesterification, the obtained product has a high purity of 95% or more. Further, the method for producing the (meth)acrylic group-containing siloxane of the present invention does not require an industrially disadvantageous purification method, and easily provides the target hydroxyl group- and (meth)acrylic group-containing siloxane with high purity.

### [Example 11]

### Production of copolymers

In a 50-mL recovery flask, were added 10 g of the hydroxyl group- and (meth)acrylic group-containing siloxane obtained in Example 1, 10 g of tetrahydrofuran, 0.1 g of N,N-dimethylformamide, and 0.002 g of azobisisobutyronitrile, and degassing was performed by bubbling using argon for 5 minutes. Then, heating was performed to 60°C and aging (polymerization) was performed for 20 hours. After aging, the reaction solution was measured with ¹H-NMR, and the polymerization rate was calculated from the peak area value of the (meth)acrylic group using N,N-dimethylformamide as the internal standard. The results are shown in Table 4.

In Examples 12 to 20 and Comparative Examples 9 to 16, Example 11 was repeated except that the compounds shown in Table 4 below were used, and copolymers were produced.

**[Table 4]**

| | Compound | Reaction solution after polymerization | Polymerization ratio, % |
|---|---|---|---|
| Example 11 | Example 1 | Colorless and transparent solution | 100 |
| Example 12 | Example 2 | Colorless and transparent solution | 98 |
| Example 13 | Example 3 | Colorless and transparent solution | 100 |
| Example 14 | Example 4 | Colorless and transparent solution | 100 |
| Example 15 | Example 5 | Colorless and transparent solution | 100 |
| Example 16 | Example 6 | Colorless and transparent solution | 100 |
| Example 17 | Example 7 | Colorless and transparent solution | 100 |
| Example 18 | Example 8 | Colorless and transparent solution | 100 |
| Example 19 | Example 9 | Colorless and transparent solution | 100 |
| Example 20 | Example 10 | Colorless and transparent solution | 97 |
| Comparative Example 9 | Comparative Example 1 | Colorless and transparent solution | 65 |
| Comparative Example 10 | Comparative Example 2 | Transparent pale yellow solution | 8 |
| Comparative Example 11 | Comparative Example 3 | No flowability, gel-like | - ^{Note 1)} |
| Comparative Example 12 | Comparative Example 4 | No flowability, gel-like | - ^{Note 1)} |
| Comparative Example 13 | Comparative Example 5 | Red transparent solution | 18 |
| Comparative Example 14 | Comparative Example 6 | No flowability, gel-like | - ^{Note 1)} |
| Comparative Example 15 | Comparative Example 7 | No flowability, gel-like | - ^{Note 1)} |
| Comparative Example 16 | Comparative Example 8 | No flowability, gel-like | - ^{Note 1)} |

| | | | |
|---|---|---|---|
| Note 1: Not soluble in heavy chloroform and not measurable | | | |

As shown in Table 4, in the polymerization reactions of Comparative Examples 9, 10, and 13, the polymerization rates were low because the polymerization inhibitor distilled into the products during vacuum distillation of the siloxanes of Comparative Examples 1, 2 and 5, inhibiting polymerization and causing the products to have low purities and many non-polymerizable compounds. In addition, the siloxanes of Comparative Examples 3, 4 and 6 to 8 had low product purity, and many of them had two (meth)acrylic groups in the same molecule that could serve as crosslinking components. Thus, in the polymerization reactions of Comparative Examples 11, 12 and 14 to 16 using these siloxanes, the solution after polymerization gelled, and the desired polymer was unable to be obtained.

In contrast, as shown in Table 4, the polymerization rates were significantly high in the polymerization reactions of Examples 11 to 20 using the siloxanes having high purities of 95% or more obtained by the method of the present invention.

That is, the siloxane compounds having hydroxyl groups and (meth)acrylic groups obtained by the present invention are useful and allows industrially disadvantageous purification processes to be minimized because there are few unreacted materials and side reactions is prevented.

### INDUSTRIAL APPLICABILITY

According to the present preparation method, siloxane compounds having hydroxyl and (meth)acrylic groups with high purity is obtained in high yields.

The present invention is not limited to the aforesaid embodiments. The aforesaid embodiments are illustrative examples, and any configuration substantially identical to the technical concept described in the claims of the present invention and which produces a similar effect is included in the technical scope of the present invention.

## Claims

1. A method for purifying a (meth)acrylic group-containing siloxane, comprising a step of subjecting a mixture comprising a (meth)acrylic group-containing siloxane represented by the following formula (I) and impurities derived from the siloxane and/or starting compounds of the siloxane to vacuum distillation in the presence of a polymerization inhibitor having one or more nitroxyl radicals in one molecule and having a molecular weight of 160 to 2,000, 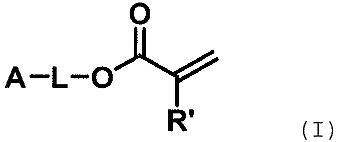 wherein R' is a hydrogen atom or a methyl group, L is a divalent hydrocarbon group having at least one hydroxyl group and optionally having an ether bond, and A is an organo(poly)siloxane residue,
to thereby remove the impurities.

2. The method for purifying the (meth)acrylic group-containing siloxane according to claim 1, wherein the purity of the (meth)acrylic group-containing siloxane represented by the said formula (I) after the purification is 95 % or more.

3. The method for purifying the (meth)acrylic group-containing siloxane according to claim 1 or 2, wherein A is represented by the following formula (II) or (II'): 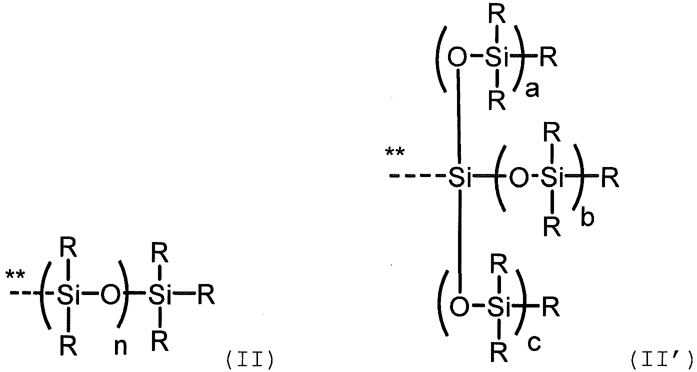 wherein R is, independently of each other, a monovalent hydrocarbon group having 1 to 6 carbons, n is an integer of 0 to 10, *a* is an integer of 0 to 10, b is an integer of 0 to 10 and c is an integer of 0 to 10, provided that a + b + c is 2 or more, the part indicated by ** is the bonding point with L in the said formula (I) and the dashed line represents the bonding hand.

4. The method for purifying the (meth)acrylic group-containing siloxane according to claim 3, wherein, in the formula (II) or (II'), R is, independently of each other, a methyl group or a butyl group, n is 4, a is 1, b is 1 and c is 0.

5. The method for purifying the (meth)acrylic group-containing siloxane according to any one of claims 1 to 4, wherein L is any one of the following formulae (1) to (4) and (1') to (4'): 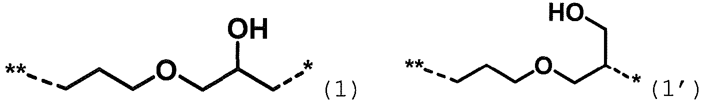 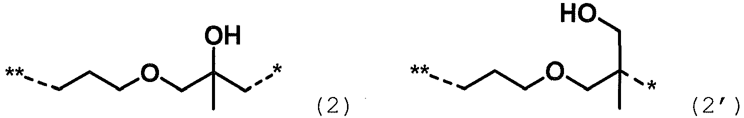 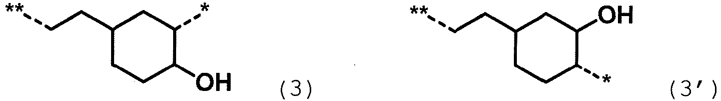 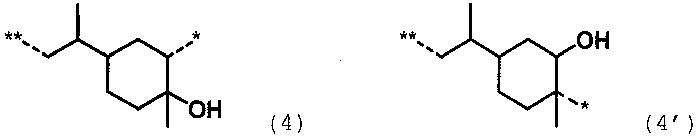 wherein the part indicated by * is the bonding point with the oxygen atom in the said formula (I), the part indicated by ** is the bonding point with A in the said formula (I) and the dashed line represents the bonding hand.

6. The method for purifying the (meth)acrylic group-containing siloxane according to any one of claims 1 to 4, wherein L is any one of the following formulae (1) to (4) and (1') to (4'): 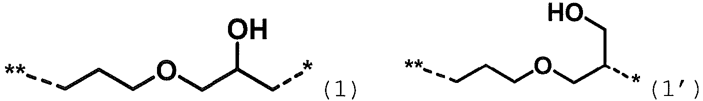 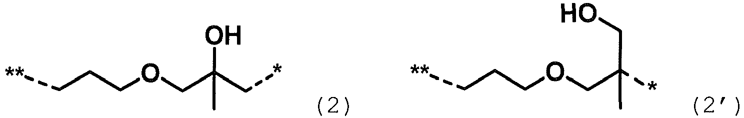 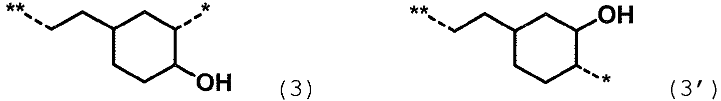 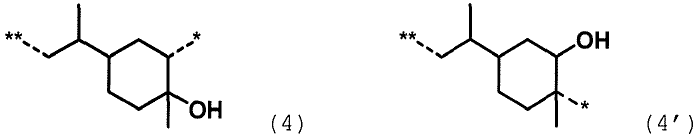 wherein the part indicated by * is the bonding point with the oxygen atom in the said formula (I), the part indicated by ** is the bonding point with A in the said formula (I) and the dashed line represents the bonding hand.

7. The method for purifying the (meth)acrylic group-containing siloxane according to any one of claims 1 to 6, wherein the polymerization inhibitor is a compound represented by the following formula (a), (a') or (b): 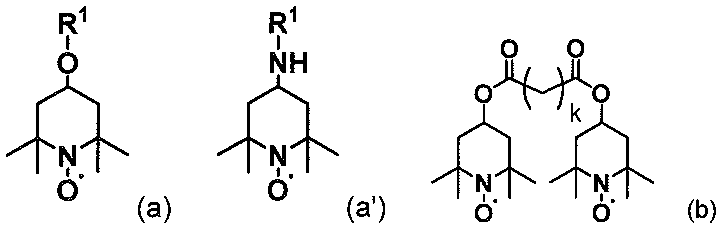 wherein R¹ is a hydrogen atom or an acyl group having 2 to 10 carbon atoms and k is an integer of 2 to 10.

8. The method for purifying the (meth)acrylic group-containing siloxane according to claim 7, wherein the polymerization inhibitor is represented by the following formula (5):

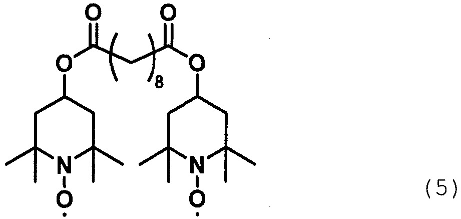

9. The method for purifying a (meth)acrylic group-containing siloxane according to any one of claims 1 to 8, wherein the impurities are one or more selected from a siloxane having two hydroxyl groups in one molecule, a siloxane having two (meth)acrylic groups in one molecule, and a siloxane having one epoxy group in one molecule.

10. The method for purifying the (meth)acrylic group-containing siloxane according to any one of claims 1 to 9, wherein the compound represented by the formula (1) is subjected to the vacuum distillation at a temperature of 160°C or more.

11. A composition comprising a (meth)acrylic group-containing siloxane represented by the following formula (I), 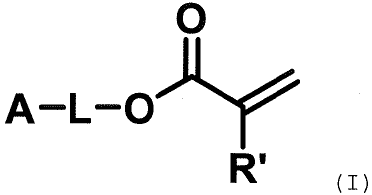 wherein R¹ is a hydrogen atom or a methyl group, L is a divalent hydrocarbon group having at least one hydroxyl group and optionally having an ether bond, and A is an organo(poly)siloxane residue,
wherein the amount of (meth)acrylic group-containing siloxane represented by formula (I) contained in the composition is 95% by mass or more, and
an amount of the following component (B), (C) or (D) in the composition is 0.5% by mass or less respectively,
(B) a siloxane having two hydroxyl groups in one molecule
(C) a siloxane having two (meth)acrylic groups in one molecule, and
(D) a siloxane having one epoxy group in one molecule.

12. The composition according to claim 11, wherein the amount of component (D) is 0 % by mass in the composition.

13. The composition according to claim 11, wherein A is represented by the following formula (II) or (II'): 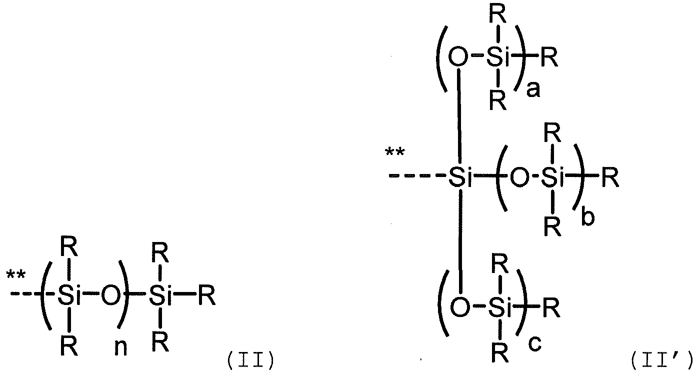 wherein R is, independently of each other, a monovalent hydrocarbon group having 1 to 6 carbons, n is an integer of 0 to 10, *a* is an integer of 0 to 10, b is an integer of 0 to 10 and c is an integer of 0 to 10, provided that a + b + c is 2 or more, the part indicated by ** is the bonding point with L in the said formula (I) and the dashed line represents the bonding hand.

14. The composition according to claim 13, wherein in the formula (II) or (II'), R is, independently of each other, a methyl group or a butyl group, n is 4, a is 1, b is 1 and c is 0.

15. The composition according to any one of claims 11 to 14, wherein L is any one of the following formulae (1) to (4) and (1') to (4'): 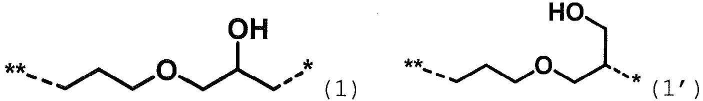 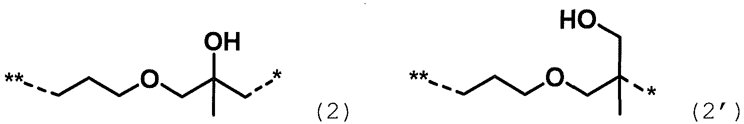 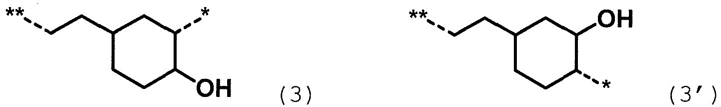 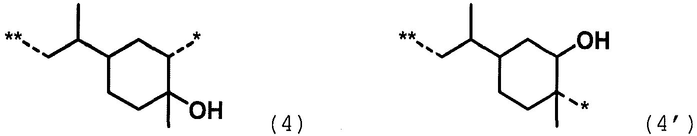 wherein the part indicated by * is the bonding point with the oxygen atom in the said formula (I), the part indicated by ** is the bonding point with A in the said formula (I) and the dashed line represents the bonding hand.

16. The composition according to claim 15, wherein the amount of the (meth)acrylic group-containing siloxane represented by the said formula (I) is 95% by mass or more in the composition and that is as a total amount of structural isomers represented by the said formula (I) in which the structure of L is different.

17. A medical material comprising the composition according to any one of claims 11 to 16.
